(19) Europäisches Patentamt
European Patent Office
Office européen des brevets

(11) Veröffentlichungsnummer: **0 307 763 A2**

# (12) EUROPÄISCHE PATENTANMELDUNG

(21) Anmeldenummer: **88114522.1**

(22) Anmeldetag: **06.09.88**

(51) Int. Cl.4: **C07C 118/00 , C07C 119/042 , C07F 7/18**

(30) Priorität: **16.09.87 DE 3730987**

(43) Veröffentlichungstag der Anmeldung: **22.03.89 Patentblatt 89/12**

(84) Benannte Vertragsstaaten: **CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Mormann, Werner, Prof. Dr.**
**Zum Wolfsloch 30**
**D-4910 Kreuztal(DE)**
Erfinder: **Leukel, Gabriele**
**Köln-Leipziger-Strasse 4**
**D-5239 Norken(DE)**

## (54) Verfahren zur Herstellung von Polyisocyanaten.

(57) Ein Verfahren zur Herstellung von Estergruppen aufweisenden Polyisocyanaten durch Umsetzung von Dicarbonsäuredichloriden mit organischen Monoisocyanaten, die eine silylierte alkoholische oder phenolische Hydroxylgruppe als Substituenten aufweisen bei -20 bis +190 °C.

EP 0 307 763 A2

## Verfahren zur Herstellung von Polyisocyanaten

Gegenstand der Erfindung ist ein Verfahren zur Herstellung von Estergruppen aufweisenden Polyisocyanaten, dadurch gekennzeichnet, daß man

a) mehrwertige Carbonsäurechloride

mit

b) organischen Monoisocyanaten, die eine silylierte alkoholische oder phenolische Hydroxylgruppe als Substituenten aufweisen und ansonsten unter den jeweiligen Reaktionsbedingungen gegenüber Isocyanat- und Chlorcarbonylgruppen inert sind,

bei -20 °C bis +190 °C zur Reaktion bringt.

Das erfindungsgemäße Verfahren stellt eine grundsätzlich neue Methode zur Herstellung von Estergruppen aufweisenden Polyisocyanaten dar, wobei die Eigenschaften der erfindungsgemäßen Verfahrensprodukte durch einfache Variation der zu ihrer Herstellung verwendeten Ausgangsmaterialien a) und/oder b) dem jeweiligen Verwendungszweck in optimaler Weise angepaßt werden können.

Für das erfindungsgemäße Verfahren a) sind beliebige mehrwertige Carbonsäurehalogenide, insbesondere Carbonsäurechloride geeignet, die gegenüber Isocyanatgruppen unter den Bedingungen des erfindungsgemäßen Verfahrens inert sind und die neben den Säurechloridgruppen keine gegenüber silylierten Hydroxylgruppen reaktionsfähige Gruppen aufweisen. Besonders gut geeignet sind Dicarbonsäuredichloride der allgemeinen Formel

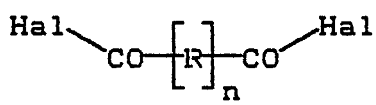

für welche

R für einen 2-wertigen aliphatischen Kohlenwasserstoffrest mit 2 bis 10 Kohlenstoffatomen oder einen gegebenenfalls gegenüber Säurechlorid- und NCO-Gruppen inerte Substituenten aufweisenden 1,2-, 1,3- oder 1,4-Phenylenrest steht, wobei im Falle des aliphatischen Kohlenwasserstoffrests zwischen den beiden Carbonylgruppen mindestens 2 Kohlenstoffatome angeordnet sind,

Hal für Chlor oder Fluor, vorzugsweise Chlor steht, und

n für 0 oder 1 steht,

Beispiele geeigneter Dicarbonsäuredichloride sind die Dichloride der Oxalsäure, Bernsteinsäure, Glutarsäure, Adipinsäure, Sebacinsäure, 1,10-Decan-dicarbonsäure, Phthalsäure, Isophthalsäure oder Terephthalsäure, wobei die letztgenannten drei Säuren in 2-Stellung inerte Substituenten wie Chlor, Brom, eine Nitrogruppe, eine Alkyl-, Alkoxy- oder Alkoxycarbonyl-Gruppe mit jeweils 1 - 20, vorzugsweise 1 - 4, Kohlenstoffatomen im Alkylrest aufweisen können, oder 4,4′-Diphenyl-dicarbonsäure. Bei diesen Dicarbonsäuredichloriden handelt es sich um altbekannte chemische Verbindungen. Ebenfalls geeignet, jedoch weniger bevorzugt, sind höherwertige Carbonsäurechloride, beispielsweise die bekannten Benzoltricarbonsäure-trichloride.

Bei der Komponente b) handelt es sich um beliebige organische Monoisocyanate, die eine silylierte alkoholische und/oder phenolische Hydroxylgruppe als Substituenten aufweisen und ansonsten unter den Reaktionsbedingungen des erfindungsgemäßen Verfahrens gegenüber Isocyanat-und Carbonylgruppen inert sind. Besonders gut geeignet sind Verbindungen der allgemeinen Formeln

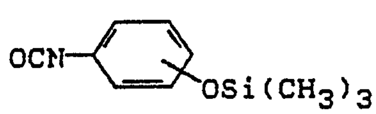

oder

$$OCN-[CH_2]_m-OSi(CH_3)_3$$

wobei
m für eine ganze Zahl von 2 bis 4 steht.

Geeignete Ausgangskomponenten b) sind beispielsweise 4-(Trimethylsilyloxy)-phenylisocyanat, 3-(Trimethylsilyloxy)-phenylisocyanat, wobei die beispielhaft genannten aromatischen Isocyanate in 2- oder 3-Stellung inerte Substituenten tragen können, wobei als Substituenten beispielsweise solche der oben im Zusammenhang mit den Phthalsäuren genannten Art in Betracht kommen, 2-(Trimethylsilyloxy)-ethylisocyanat, 3-(Trimethylsilyloxy)-propylisocyanat oder 4-(Trimethylsilyloxy)-butylisocyanat. Ebenfalls geeignet, jedoch weniger bevorzugt, sind die entsprechenden Verbindungen deren Substituenten am Silicium zumindest teilweise höhere Alkylreste, beispielsweise Ethyl-, Propyl- oder Butyl-Reste anstelle der Methylreste darstellen.

Die Herstellung der silylierten Ausgangsverbindungen b) gelingt beispielsweise durch Umsetzung der den Isocyanaten entsprechenden Aminoalkohole mit Bis-(4-chlorphenyl)-carbonat zum N-(2-Hydroxyalkyl))-carbamidsäure-4-chlorphenylester und dessen anschließender Silylierung mit Trimethylchlorsilan in Gegenwart einer äquimolaren Menge an Triethylamin (vgl. z.B. H.R. Kricheldorf, Liebigs Annalen der Chemie (1973), 772).

Eine weitere Möglichkeit der Herstellung der Ausgangskomponenten b) besteht in der thermischen Spaltung des dem Monoisocyanat entsprechenden O-Phenyl-urethans (vgl. z.B. H.R. Kricheldorf a.a.O.).

Eine weitere Möglichkeit der Herstellung der Ausgangskomponenten b) besteht schließlich in der Umsetzung von, den Monoisocyanaten entsprechenden, O-silylierten Aminophenolen oder Aminoalkoholen, insbesondere solchen der Formeln

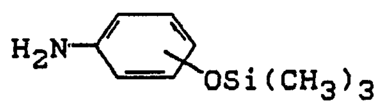

oder

$$H_2N-[CH_2]_m-OSi(CH_3)_3$$

mit einem hohen Überschuß an hochsiedenden Polyisocyanaten wie beispielsweise 4,4′-Diisocyanatodiphenylmethan, wobei diese Umsetzung bei Temperaturen von bis zu 200 °C, oftmals am Ende unter vermindertem Druck und die Gewinnung des als Ausgangsmaterial b) einsetzbaren Monoisocyanats in Form des bei dieser Umsetzung anfallenden Destillats erfolgen.

Die Herstellung der bei dieser Umsetzung eingesetzten O-silylierten Aminophenole bzw. Aminoalkohole der zuletztgenannten Formeln kann beispielsweise in Analogie zu der von E. Lukevits u.a., Zh. Obshch. Khim. 39, 806 (1969) beschriebenen Methode durch Monosilylierung der Aminophenole bzw. Aminoalkohole unter Verwendung von Hexamethyldisilazan (HMDS) erfolgen. Die Umsetzung kann beispielsweise dergestalt erfolgen, daß man die Aminophenole bzw. Aminoalkohole mit HMDS im Molverhältnis von 1:0,5 bis 1:0,6 in Gegenwart einer katalytischen Menge an Trimethylchlorsilan auf Temperaturen von bis zu 170 °C, vorzugsweise 50 bis 170 °C erwärmt, wobei die selektive Silylierung der Hydroxylgruppen unter Ammoniakabspaltung erfolgt.

Bei der Durchführung des erfindungsgemäßen Verfahrens wird die Menge der Reaktionspartner a) und b) im allgemeinen so gewählt, daß auf jedes Mol an Chlorcarbonylgruppen der Komponente a) 0,8 bis 1,2, vorzugsweise 1 Mol an silylierten Hydroxylgruppen der Komponenten b) entfallen. Die Verwendung eines größeren Überschusses einer der beiden Komponenten wäre zwar möglich, würde jedoch lediglich zu Ausbeuteverlusten führen.

Als Nebenprodukt entsteht bei der erfindungsgemäßen Umsetzung das entsprechende Chlorsilan, das leicht durch Destillation abgetrennt werden kann.

Die erfindungsgemäß Umsetzung erfolgt vorzugsweise innerhalb des Temperturbereichs von 20 bis 180 °C, insbesondere von 60 bis 160 °C, wobei man das Ende der Reaktion (bei Verwendung von maximal äquivalenten Mengen an Säurechloriden) am Verschwinden der Säurechlorid-Carbonylbande bei 1800 $cm^{-1}$ im Infrarotspektrum leicht erkennen kann.

Die Reaktion in Ab- oder Anwesenheit eines geeigneten Lösungsmittels durchgeführt werden. Geeignete Lösungsmittel sind beispielsweise Diethylether, Toluol, Xylol, Chlorbenzol, o-Dichlorbenzol, Trichlorethylen, Essigsäureethylester, Essigsäurebutylester oder beliebige Gemische derartiger Lösungsmittel.

Die erfindungsgemäßen Verfahrensprodukte stellen Estergruppen aufweisende Diisocyanate dar, deren

Eigenschaften, wie bereits erwähnt, durch Variation der Ausgangskomponenten a) und b) dem jeweils nagestrebten Verwendungszweck optimal angepaßt werden können. Die erfindungsgemäßen Verfahrensprodukte mit aliphatisch gebundenen Isocyanatgruppen eignen sich insbesondere zur Herstellung von hochwertigen Ein- oder Zweikomponenten-Polyurethanlacken. Die erfindungsgemäßen Verfahrensprodukte mit aromatisch gebundenen Isocyanatgruppen eignen sich beispielsweise zur Herstellung von Polyurethanelastomeren nach dem Polyisocyanat-Polyadditionsverfahren, wobei die erfindungsgemäßen Verfahrensprodukte die hierfür üblicherweise eingesetzten Diisocyanate ganz oder teilweise ersetzen können. Auf aromatischem Dicarbonsäuredichlorid basierende erfindungsgemäße Verfahrensprodukte weisen oftmals interessante LC-Eigenschaften auf.

In den nachfolgenden Ausführungsbeispielen beziehen sich alle Prozentangaben auf Gewichtsprozente.

Herstellung von in den nachstehenden Beispielen eingesetzten Ausgangsmaterialien b)

1. O-silylierte Aminophenole bzw. Aminoalkohole (allgemeine Herstellungsvorschrift)

In einem ausgeheizten 500 ml Dreihalskolben mit Magnetrührstab, Innenthermometer, Tropftrichter und Rückflußkühler mit Blasenzähler, Trockenrohr und Gasableitung werden 1 Mol Aminophenol bzw. Aminoalkohol vorgelegt und 0,55 Mol Hexamethyldisilazan zügig zugetropft. Nach Zugabe von drei Tropfen Trimethylmonochlorsilan als Katalysator wird die in manchen Fällen heterogene Flüssigkeitsmischung langsam erwärmt. Bei 50 bis 55 °C kann die erste Ammoniakentwicklung beobachtet werden, die bei den Aminoalkoholen bei 85 bis 95 °C und den Aminophenolen bei 130 °C sehr heftig wird. Die Temperatur wird bis zum Ende der Gasentwicklung (drei bis sieben Stunden) auf 140 bis 170 °C gesteigert. Dann wird die homogene Flüssigkeit zur Reinigung über eine verspiegelte Vigreuxkolonne rektifiziert.

1.1 4-Trimethylsiloxyphenylamin

Ausbeute: 88,6 %
Kp.: 114 -116 °C (20 mbar)
$n_D^{20}$ : 1,5189

1.2 3-Trimethylsiloxyphenylamin

Ausbeute: 81 %
Kp.: 115 - 116 °C (20mbar)

1.3 4-Trimethylsiloxybutylamin

Ausbeute: 83 %
Kp.: 63 - 64 °C (20 mbar)
IR-Spektrum: 3380 und 3300 $cm^{-1}$ (NH-Valenzschwingung)
1600 $cm^{-1}$ (NH-Deformationsschwingung)

Elementaranalyse (%):

Berechnet:
C 51,44 H 11, 6 N 6,0
Gefunden:
C 51,40 H 11,1 N 5,8

1.4 3-Trimethylsiloxypropylamin

Ausbeute: 75 %
Kp.: 134 - 135 °C (495 mbar)
$n_D^{20}$ : 1,4195
IR-Spektrum: 3390 und 3340 $cm^{-1}$ (NH-Valenzschwingung)
1600 $cm^{-1}$ (NH-Deformationsschwingung)

| | | | | |
|---|---|---|---|---|
| $^1$H-NMR-Spektrum: | 3,45 (t); 2 : | 2,55 (t); 2 : | 1,45 (m); 2 : | 0,85 ppm (s) 2 |

1.5 2-Trimethylsiloxyethylamin

Ausbeute: 83 %
Kp.: 38 - 39 °C (20 mbar)
$n_D^{20}$ : 1,4131
IR-Spektrum: 3380 und 3300 $cm^{-1}$ (NH-Valenzschwingung)
1590 $cm^{-1}$ (NH-Deformationsschwingung)

1.6 2-Trimethylsiloxy-2-methylpropylamin

Ausbeute: 85 %
Kp: 38 - 38,5 °C (20 mbar)
$n_D^{20}$ : 1,4089
IR-Spektrum: 3380 und 3280 $cm^{-1}$ (NH-Valenzschwingung)
1590 $cm^{-1}$ (NH-Deformationsschwingung)

2. O-silylierte Isocyanatophenole bzw. Isocyanatoalkanole (allgemeine Herstellungsvorschrift)

In einem 500 ml Planschliffkolben mit Magnetrührstab, Innenthermometer, Tropftrichter und Destillationsbrücke, sowie einem 100 ml Stickstoffkolben als Vorlage werden 500 g (2 Mol) 4,4′-Diisocyanatodiphenylmethan eingewogen und unter Argon aufgeschmolzen. Bei 110 bis 120 °C werden 0,4 Mol Trimethylsiloxyaryl(alkyl)amin langsam zugetropft, wobei sich weiße Nebel bilden. Nach beendeter Zugabe des Amins wird die Apparatur unter weiterem Aufheizen bis auf 200 °C vorsichtig evakuiert. Die überdestillierende farblose Flüssigkeit wird anschließend fraktioniert.

2.1 4-Trimethylsiloxyphenylisocyanat

Ausbeute: 95 %
Kp.: 52 - 53 °C (0,24 mbar)
$n_D^{20}$ : 1,5032
IR-Spektrum: 2280 $cm^{-1}$ (N = C = O)

| | | |
|---|---|---|
| $^1$H-NMR-Spektrum: | 6,92 (m); 4 : | 0,31 ppm (s) 9 |

Elementaranalyse (%):

Berechnet:
C 58,0 H 6,3 N 6,8
Gefunden:
C 58,4 H 6,2 N 7,2

2.2 3-Trimethylsiloxyphenylisocyanat

Ausbeute: 79 %
Kp.: 109 °C (20 mbar)
$n_D^{20}$ : 1,5025
IR-Spektrum: 2280 $cm^{-1}$ (N=C=O)

| | | |
|---|---|---|
| $^1$H-NMR-Spektrum: | 6,93 (m); 4 : | 0,34 ppm (s) 9 |

Elementaranalyse (%)
Berechnet:
C 58,0 H 6,3 N 6,8
Gefunden:
C 57,9 H 6,4 N 6,7

2.3 4-Trimethylsiloxybutylisocyanat

Ausbeute: 70 %
Kp.: 75 - 76 °C (20 mbar)
$n_D^{20}$ : 1,4250
IR-Spektrum: 2270 $cm^{-1}$ (N=C=O)

| | | | | |
|---|---|---|---|---|
| $^1$H-NMR-Spektrum: | 3,58 (t); 2 : | 3,27 (t); 2 : | 1,58 (m); 4 : | 0.05 ppm (s) 9 |

Elementaranalyse (%):

Berechnet:
C 51,3 H 9,1 N 7,5
Gefunden:
C 50,4 H 9,0 N 7,4

2.4 3-Trimethylsiloxypropylisocyanat

Ausbeute: 94 %
Kp.: 68,5 - 69 °C (20 mbar)
$n_D^{20}$ : 1,4200
IR-Spektrum: 2280 $cm^{-1}$ (N=C=O)

| | | | | |
|---|---|---|---|---|
| $^1$H-NMR-Spektrum: | 3,65 (t); 2 : | 3,39 (t); 2 : | 1,78 (m); 2 : | 0,09 ppm (s) 9 |

Elementaranalyse (%):

Berechnet:
C 48,5 H 8,7 N 8,1
Gefunden:
C 48,9 H 9,7 N 8,2

2.5 2-Trimethylsiloxyethylisocyanat

Ausbeute: 84 %
Kp.: 45 °C (20 mbar)
$n_D^{20}$ : 1,4140
IR-Spektrum: 2280 und 2240 $cm^{-1}$ (N=C=O)

| | | | |
|---|---|---|---|
| $^1$H-NMR-Spektrum: | 3,68 (t); 2 : | 3,30 (t); 2 : | 0,17 (s) ppm 9 |

Elementaranalyse (%):

Berechnet:
C 45,2 H 8,2 N 8,8
Gefunden:
C 44,7 H 7,6 N 8,8

2.6 2-Trimethylsiloxy-2-methylpropylisocyanat

Ausbeute: 96%
Kp.: 57 °C
$n_D^{20}$ : 1,4011
IR-Spektrum: 2260 $cm^{-1}$ (N=C=O)

| | | | |
|---|---|---|---|
| $^1$H-NMR-Spektrum: | 3,40 (s); 2 : | 1,22 (s); 6 : | 0,12 ppm (s) 9 |

Elementaranalyse (%):

Berechnet:
C 51,3 H 9,2 N 7,5
Gefunden:
C 51,9 H 9,3 N 7,6

3. Erfindungsgemäßes Verfahren unter Verwendung von Oxalylchlorid als Komponente a) (allgemeine Vorschrift)

In einem ausgeheizten 25 ml Rundkolben mit Magnetrührstab, Mikrodestillationsbrücke, Trockenrohr und Vorlage werden 80 mMol Trimethylsiloxyalkylisocyanat mit 40 mMol Oxalylchlorid unter Zugabe von vier bis fünf Kristallen 4-Dimethylaminopyridin als Katalysator versetzt. Das Reaktionsgemisch wird bei 80 bis 90 °C gerührt. Nach 11 bis 24 Stunden ist die Reaktion beendet, und das Produkt kann an der Kugelrohrdestillationsanalage gereinigt werden.

Nach diesem Verfahren wurden die nachstehenden Diisocyanate hergestellt:

3.1 Oxalsäure-bis-(4-isocyanatobutylester)

Ausbeute: 74%
Kp.: 190 - 2120 °C ($1,2.10^{-5}$ mbar)
$n_D^{20}$ : 1,4660
IR-Spektrum: 2280 $cm^{-1}$ (N=C=O)
1770 und 1750 $cm^{-1}$ (CO)

| | | | |
|---|---|---|---|
| $^1$H-NMR-Spektrum: | 4,20 (t); 2 : | 3,30 (t); 2 : | 1,70 (m) ppm 4 |

Elementaranalyse (%): Berechnet:
C 50,7 H 5,7 N 9,9
Gefunden:
C 50,8 H 5,8 N 10,1

3.2 Oxalsäure-bis-(3-isocyanatopropylester)

Das Produkt konnte nicht destilliert werden, da es bereits im Vakuum ($1.10^{-4}$ mbar) bei 130 °C Zersetzungserscheinungen zeigte.
IR-Spektrum: 2280 $cm^{-1}$ (N=C=O)
1765 und 1740 $cm^{-1}$ (CO)
Elementaranalyse (%):
Berechnet:
C 46,9 H 4,7 N 10,9
Gefunden:
C 46,2 H 4,8 N 11,9

4. Erfindungsgemäßes Verfahren unter Verwendung von Adipinsäuredichlorid als Komponente a) (allgemeine Vorschrift)

In einem ausgeheizten 25 ml Rundkolben mit Magnetrührstab, Mikrodestillationsbrücke, Trockenrohr und Vorlage werden zu 80 mMol Trimethylsiloxyalkylisocyanat 40 mMol Adipinsäuredichlorid hinzugefügt. Unter Rühren wird die Flüssigkeit auf 110 bis 130 °C erwärmt, wobei das gebildete Trimethylchlorsilan abdestilliert. Nach 24 bis 29 Stunden ist die Reaktion beendet, und das Produkt kann an der Kugelrohrdestillationsanlage gereinigt werden.

Nach dieser Vorschrift wurden die nachstehenden Diisocyanate hergestellt:

4.1 Adipinsäure-bis-(4-isocyanatobutylester)

Ausbeute: 72%
Kp.: 190 - 200 °C (0,027 mbar)
$n_D^{20}$ : 1,4670
IR-Spektrum: 2280 $cm^{-1}$ (N = C = O)
1735 $cm^{-1}$ (CO)

| | | | | |
|---|---|---|---|---|
| ¹H-NMR-Spektrum: | 4,05 (t); 4 : | 3,34 (t); 4 : | 2,31 (t); 4 : | 1,66 (m) ppm 12 |

Elementaranalyse (%):
Berechnet:
C 56,5 H 7,1 N 8,2
Gefunden:
C 56,3 H 7,2 N 8,3

4.2 Adipinsäure-bis-(3-isocyanatopropylester)

Ausbeute: 61 %
Kp.: 170 - 190 °C ($7.10^{-6}$ mbar)
$n_D^{20}$ : 1,4679
IR-Spektrum: 2280 $cm^{-1}$ (N = C = O)
1735 $cm^{-1}$ (COO)
¹H-NMR-Spektrum: 4,08 (t); 3,36 (t); 2,25 (m); 1,87 (m); 1,59 (m) ppm im Verhältnis 4:4:4:4:4
Elementaranalyse (%)
Berechnet:
C 53,8 H 6,4 N 9,0
Gefunden:
C 54,0 H 5,9 N 9,1

5. Erfindungsgemäßes Verfahren unter Verwendung von Isophthalsäure- und Terephthalsäuredichlorid als Komponente a) (allgemeine Vorschrift)

In einem ausgeheizten 25 ml-Kolben mit Mikrodestillationsaufsatz werden 0,025 Mol des Isocyanatosäurechlorids und die äquimolare Menge an Trimethylsiloxyisocyanat vorgelegt. Als Lösungsmittel werden 6 ml o-Dichlorbenzol hinzugefügt und die Reaktionsmischung mit dem betreffenden Katalysator auf 165 °C für nicht länger als 24 Stunden erwärmt, wobei das gebildete Trimethylchlorsilan abdestilliert.

Zur Aufarbeitung und Reinigung wird das betreffende Esterdiisocyanat aus dem geeigneten Lösungsmittel umkristallisiert.

| Nr. | Diisocyanat | Kat. | umkristallisiert aus | Ausbeute % | Schmelzverhalten in °C 2) | IR-Banden in $cm^{-1}$ | Elementaranalyse % ber: | gef: |
|---|---|---|---|---|---|---|---|---|
| 5.1 | 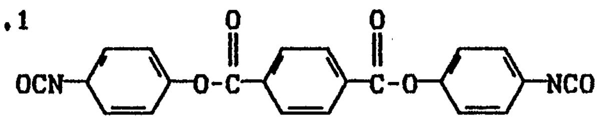 | DMAP 1) <br> $TiCl_3$ | o-Dichlorbenzol | 34 <br> 30 | K 180 n)300d$^c$ | 2340+2300,1730 <br> 2280, 1730 | C:66,0 <br> H: 3,0 <br> N: 7,0 | 65,8 <br> 2,95 <br> 6,85 |
| 5.2 | 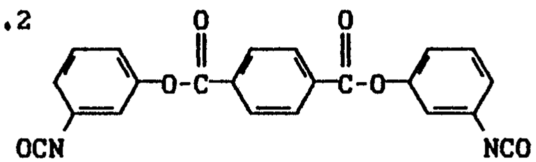 | DMAP | Essigester | 78 | K 148 i | 2310+2290, 1740 | " | 65,6 <br> 3,1 <br> 7,1 |
| 5.3 | 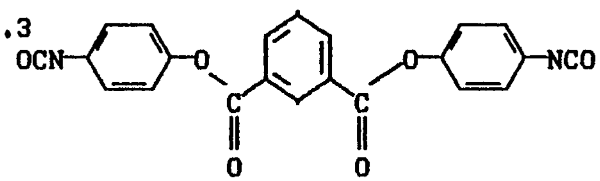 | DMAP <br> $TiCl_3$ | Heptan | 81 <br> 85 | K 160a)300d$^c$ | 2310, 1735 | " | 65,8 <br> 3,5 <br> 7,1 |
| 5.4 | 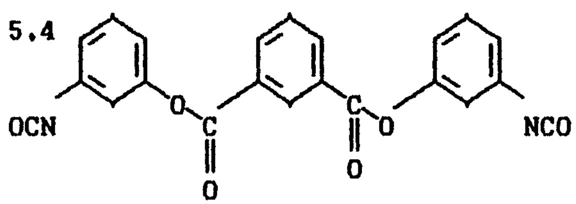 | DMAP | Heptan | 76 | K 110-117 i | 2300, 1740 | " | 65,5 <br> 3,1 <br> 6,9 |
| 5.5 | 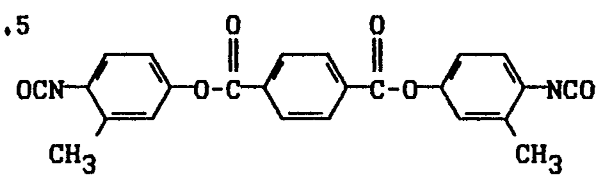 | $H_2SO_4$ | o-Dichlorbenzol | 50 | K 180 a 250; | 2305, 1725 | | |

1) 4-Dimethylaminopyridin
2) d$^c$: Zersetzung im anisotropen Bereich, K = Kristallin, n = nematisch, i = isotrop, a = anisotrop

## Ansprüche

1. Verfahren zur Herstellung von Estergruppen aufweisenden Polyisocyanaten, dadurch gekennzeichnet, daß man

a) Dicarbonsäuredichloride

mit

b) organischen Monoisocyanaten, die eine silylierte alkoholische oder phenolische Hydroxylgruppe als Substituenten aufweisen und ansonsten unter den jeweiligen Reaktionsbedingungen gegenüber Isocyanat- und Chlorcarbonylgruppen inert sind,

bei -20 °C bis +190 °C zur Reaktion bringt.

2. Verfahren gemäß Anspruch 1, dadurch gekennzeichnet, daß man als Komponente a) Verbindungen der allgemeinen Formel

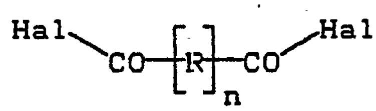

verwendet, für welche

R für einen 2-wertigen aliphatischen Kohlenwasserstoffrest mit 2 bis 10 Kohlenstoffatomen oder einen 1,2-, 1,3- oder 1,4-Phenylenrest steht, wobei im Falle des aliphatischen Kohlenwasserstoffrests zwischen den beiden Carbonylgruppen mindestens 2 Kohlenstoffatome angeordnet sind,

Hal für Chlor oder Fluor, vorzugsweise Chlor steht, und

n für 0 oder 1 steht.

3. Verfahren gemäß Anspruch 1 und 2, dadurch gekennzeichnet, daß man als Komponente b) Verbindungen der Formeln

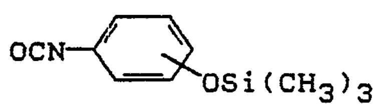

oder

$$OCN-[CH_2]_m-OSi(CH_3)_3$$

verwendet, wobei

m für eine ganze Zahl von 2 bis 4 steht.